# DEMANDE DE BREVET EUROPEEN

(11) **EP 0 570 301 A1**
(43) Date de publication de la demande: **18.11.1993**
(21) Numéro de dépôt: 93401249.3
(22) Date de dépôt: 14.05.1993
(51) Int. Cl.: A61B 17/36

(54) **Dispositif cryogénique portable**

(30) Priorité: 15.05.1992 FR 9205938
(71) Demandeur: Harari, Albert, F-33500 Libourne (FR)
(72) Inventeur: Harari, Albert, F-33500 Libourne (FR)

(57) **Abrégé**

L'invention concerne un dispositif à main pour le traitement cryogénique d'un corps, comportant un conteneur pour un fluide cryogénique et une extrémité d'application.

Selon l'invention, le conteneur est en forme de réservoir isotherme (1,1',50,500) comportant à l'une de ses extrémités une large ouverture de remplissage obturable par un bouchon (6 à 10,65,650) tandis que son autre extrémité est traversée par un canal ou conduit (12,12',54,540) d'écoulement du liquide, cette autre extrémité étant équipée d'un embout d'application (5,5',58, 84,580) adapté, le bouchon d'obturation du réservoir étant pourvu d'un évent (11a,72,720), d'échappement de la fraction vaporisée du liquide réfrigérant et comportant le cas échéant, des moyens (10, 10a) pour régler la section de cet évent.

L'invention trouve notamment application dans la cryothérapie et cryochirurgie dans le domaine médical et vétérinaire.

## Description

La présente invention concerne des dispositifs cryogéniques de construction simple et de tailles réduites, tenant dans le creux d'une main, permettant de recueillir, de transporter et d'appliquer, en exerçant éventuellement une pression mécanique, une quantité modulable de gaz liquéfié cryogénique, sur tout matériau que l'on désire refroidir ou congeler.

En médecine et dans l'exercice de l'art vétérinaire, la congélation est fréquemment utilisée pour la conservation, le diagnostic et le traitement de tissus sains ou pathologiques.

La neige carbonique -78° C et le protoxyde d'azote -84° C sont utilisés mais actuellement plutôt délaissés au profit de l'azote liquide, plus efficace, grâce à sa très basse température d'ébullition : -196° C.

Les conditions matérielles de l'utilisation de l'azote liquide en médecine présentent, à ce jour, deux lacunes majeures :
- le matériel courant n'offre pas les garanties nécessaires et suffisantes d'asepsie que son usage impose en médecine ;
- lorsqu'il offre une bonne sécurité d'asepsie, il comporte en contre-partie une lourdeur de manipulation, un encombrement et un coût, qui exercent un frein indiscutable à la diffusion de la cryothérapie, à savoir l'utilisation de la congélation en thérapeutique et en cryochirurgie, c'est-à-dire l'utilisation de la congélation pour la destruction de masses tumorales bénignes ou malignes.

Les praticiens disposent habituellement, pour effectuer les gestes courants de cryothérapie, d'une bonbonne isotherme dans laquelle sont conservés 10 à 30 litres d'azote liquide et dans laquelle ils puisent une petite quantité. de l'ordre de 5 à 50 millilitres, à l'aide d'un godet serti sur une tige métallique de 50 centimètres de longueur, au moment de son utilisation.

L'azote nécessaire est alors prélevé dans le godet au moyen d'un porte-coton confectionné par le praticien puis appliqué sur la lésion.

Cette manipulation est réalisée plusieurs fois au cours d'une même séance de traitement. Or, il est de connaissance récente que d'éventuelles particules virales, prélevées lors de l'application du coton sur des lésions pouvant comporter des virus, puis ramenées dans le godet au moment de la réimprégnation, sont susceptibles de contaminer le réservoir principal dans lequel elles ne seraient pas détruites.

Bien que le pouvoir contaminant de ces virus congelés n'ait pas été prouvé, il apparaît tout de même indispensable de mettre au point des systèmes d'application de l'azote liquide ou de tout autre liquide réfrigérant, qui évitent tout risque de transmission virale.

On a déjà proposé des équipements qui évitent l'utilisation du porte-coton et l'éventuelle contamination, de ce fait, du réservoir principal.

Dans cette catégorie de matériel cryogénique selon l'art antérieur (la demande de brevet français n° 2.353.014), un jet d'azote liquide ou gazeux est projeté sur la zone à traiter à partir d'une source autopressurisante et autostabilisante du fluide de refroidissement saturé à la chaleur.

Dans ce matériel, le fluide réfrigérant est stocké dans un réservoir stationnaire de grande capacité ou dans un récipient manuel de type vase de DEWAR, d'une capacité de 500 millilitres, permettant une autonomie de 6 à 8 heures environ.

L'inconvénient de ce système est de ne pas permettre d'appliquer l'azote sous pression mécanique, nécessaire à la pénétration en profondeur du froid et d'entraîner une importante déperdition d'azote liquide qui coule et se disperse de façon incontrôlée, rendant très délicate voire impossible le traitement de zones péri-orificielles. De plus, ce type de dispositif ne fonctionne qu'en position verticale.

Dans une autre catégorie de matériel selon l'art antérieur, le courant de vapeur et de fluide réfrigérant est confiné dans un passage d'écoulement pourvu typiquement d'un coude de retour formé dans un matériau très bon conducteur de la chaleur, avec un évent vers l'atmosphère en un point éloigné.

L'élément échangeur de calories peut être placé près ou au contact de la surface à refroidir, sans contact direct entre le liquide réfrigérant et cette surface.

Cet échangeur est donc propre et sa conception permet l'appui et donc la congélation en profondeur, mais, relié par une tubulure au réservoir principal, lequel est à son tour, relié à une bonbonne d'azote gazeux servant à la pressurisation de l'azote liquide, il constitue un matériel lourd, statique, encombrant et coûteux, caractéristiques pénalisantes pour le développement de la méthode cryochirurgicale.

On reconnaît que le contact direct du liquide réfrigérant, à la condition qu'il soit propre, avec la lésion, est un gage d'efficacité et qu'il est nécessaire, le plus souvent, d'exercer un appui pour permettre une congélation en masse.

Pour éviter les inconvénients des dispositifs selon l'art antérieur et permettre la mise en application des caractéristiques sus-citées, l'invention propose un petit réservoir isotherme manuel et autonome, qui permette de recueillir la quantité de liquide réfrigérant nécessaire au traitement d'un cas et de l'appliquer directement sur le corps ou la zone à congeler, en exerçant au besoin, l'appui nécessaire à la congélation du volume cible.

Plus précisément, le dispositif selon l'invention est constitué d'un petit réservoir isotherme comportant à l'une de ses extrémités une large ouverture de remplissage obturable par un bouchon tandis que son autre extrémité est traversée par un conduit ou canal d'écoulement du liquide, cette autre extrémité étant équipée d'un embout d'application adapté, le bouchon d'obturation du réservoir étant pourvu d'un évent d'échappement de la fraction vaporisée du liquide réfrigérant et comportant le cas échéant, des moyens pour régler la section de cet évent.

Le liquide ne peut pas s'écouler par le conduit ou canal prévu à l'extrémité du réservoir, lorsque d'une part, le dispositif est muni de l'embout d'application et d'autre part, l'évent d'échappement reste ouvert, en particulier pour un canal d'une section de l'ordre de 1 millimètre. Le liquide s'écoulera lorsque l'évent d'échappement sera obturé, c'est-à-dire lorsque la fraction vaporisée du liquide réfrigérant sera comprimée. Ce canal ou conduit peut, dans certains modes de réalisation, être de section très petite, à savoir de l'ordre du dixième de millimètre, on parle alors de canal capillaire. La section du canal capillaire permet au liquide réfrigérant de ne pas s'écouler lorsque l'évent d'échappement reste ouvert et de s'écouler lorsque l'évent d'échappement est obturé, même lorsque le dispositif ne comporte pas d'embout d'application.

Deux versions du dispositif sont prévues :
- Une version destinée à l'usage unique, soit monobloc (embout compris) soit en deux pièces où l'embout vient se fixer sur le réservoir par tout moyen approprié tel que vissage ou encastrement, cette version étant réalisée en matériaux léger, peu coûteux et non polluants et conditionnée en sachet stérile.
- Une version destinée à être réutilisée, dans laquelle le réservoir est réalisé en matériaux résistants et stérilisables, l'embout applicateur rapporté (vissé ou encastré) pouvant être dans ce cas à usage unique jetable ou réutilisable et en matériaux capables d'être stérilisés.

Au moment du geste cryogénique, le praticien choisit dans une panoplie de plusieurs réservoirs (de 5 à 100 millilitres, par exemple : 5 ml, 10 ml, 20 ml, 50 ml, 100 ml), celui qui correspond le mieux au besoin du moment. 11 le remplit au moyen du godet dont il dispose déjà et sur lequel il aura adapté un bec verseur, en puisant dans le réservoir principal d'azote liquide, ou au moyen d'un entonnoir.

Il choisit ensuite un embout, adapté à l'usage qu'il s'apprête à en faire, dans une deuxième panoplie qui propose différentes tailles et formes, comme décrit plus loin. Il fixe cet embout sur le réservoir en le vissant ou en l'encastrant ou par tout moyen analogue.

Enfin, il adapte sur le réservoir le bouchon encastrable ou vissant pour le modèle aussi bien jetable que réutilisable.

Le praticien dispose alors d'un outil autonome qui lui permet de se déplacer et au besoin de quitter la salle dans laquelle se trouve le réservoir principal et qu'il pourrait au besoin partager avec d'autres praticiens.

Dans le réservoir partiellement rempli, se trouvent une phase d'azote liquide et une phase au-dessus, d'azote gazeux.

Le réservoir isotherme est percé à son extrémité inférieure, ainsi que l'embout applicateur, mais l'azote liquide ne s'écoule par l'orifice de fin calibre du réservoir que lorsque l'azote gazeux se trouve comprimé, du fait de l'obturation par le bouchon.

Le bouchon est doté d'un évent dont l'ouverture ou la fermeture par une simple pression d'un doigt, permet de réguler simplement et efficacement la distribution de l'azote liquide par l'embout applicateur (ou embout d'application), au moment du traitement.

L'embout applicateur se vissant, ou s'encastrant, stérile, reçoit l'azote liquide du réservoir, le filtre, l'accumule dans sa partie inférieure poreuse et le vaporise au contact de la surface à refroidir, sur laquelle il peut appuyer plus ou moins fortement.

En conséquence, c'est un premier objet de l'invention de fournir un dispositif léger, autonome, permettant de traiter avec une seule charge toutes les lésions d'un même patient, sans risque de contamination virale et avec un gain de temps appréciable.

Un autre objet de l'invention est de procurer un procédé amenant le liquide réfrigérant au contact de la surface à refroidir, puisqu'il a été prouvé que l'efficacité de la cryothérapie est proportionnelle à la vitesse du refroidissement. L'invention prévoit toutefois une variante de l'embout applicateur évitant le contact direct du liquide réfrigérant avec une partie qui doit être assurée d'une stérilité totale.

Un autre objet de l'invention est de permettre par la conception de son embout applicateur, l'appui sur la lésion à traiter et donc une pénétration du froid en profondeur mais aussi d'autoriser par l'utilisation d'un gel contact, l'adhérence au volume cible et donc une traction de ce volume, lorsqu'il est nécessaire de le dégager d'une zone fragile sous-jacente.

Un autre objet de l'invention est de permettre, grâce à son bouchon à évent, de moduler avec précision, la quantité de liquide réfrigérant à distribuer et de tenir l'appareil en position verticale ou inclinée.

Un autre objet de l'invention est de procurer un procédé économique en liquide réfrigérant et en matériel.

Un autre objet de l'invention est de s'adapter aux besoins spécifiques de différents secteurs, médicaux ou autres.

Le gaz liquéfié communément utilisé est l'azote liquide mais tout autre gaz réfrigérant, à la condition qu'il soit compatible avec le secteur d'application, est susceptible d'être utilisé avec la présente invention.

L'invention sera mieux comprise et d'autres but, caractéristiques, détails et avantages de celle-ci apparaîtront plus clairement au cours de la description explicative qui va suivre, faite en référence aux dessins annexés, dans lesquels :
- la figure 1 est une coupe du dispositif dans sa version à usage unique, comprenant un réservoir avec son isolation, le bouchon encastrable avec son évent et un embout vissant ;
- la figure 2 est une vue en perspective du dispositif dans sa version destinée à l'usage unique et permettant de mieux comprendre la forme du bouchon ;
- la figure 2a est une coupe détaillée de l'embout vissant applicateur ;
- la figure 2b est une variante de cet embout à extrémité large ;
- la figure 3 est une coupe d'ensemble du dispositif dans sa version réutilisable ;
- la figure 4 est une coupe agrandie partielle de la figure 3 ;
- les figures 5, 6 et 7 sont des coupes de variantes de l'embout applicateur ;
- la figure 8 est une coupe du dispositif suivant un autre mode de réalisation de l'invention où l'embout applicateur est à usage unique et est encastré dans le réservoir réutilisable ;
- la figure 9 est une coupe d'une variante de la zone de connexion de l'embout avec le réservoir, représentée à la figure 8 ; et
- la figure 10 est une coupe du dispositif suivant un mode de réalisation où l'embout d'application est à usage unique et où l'extrémité distale du réservoir réutilisable est encastré dans l'extrémité proximale de l'embout d'application.

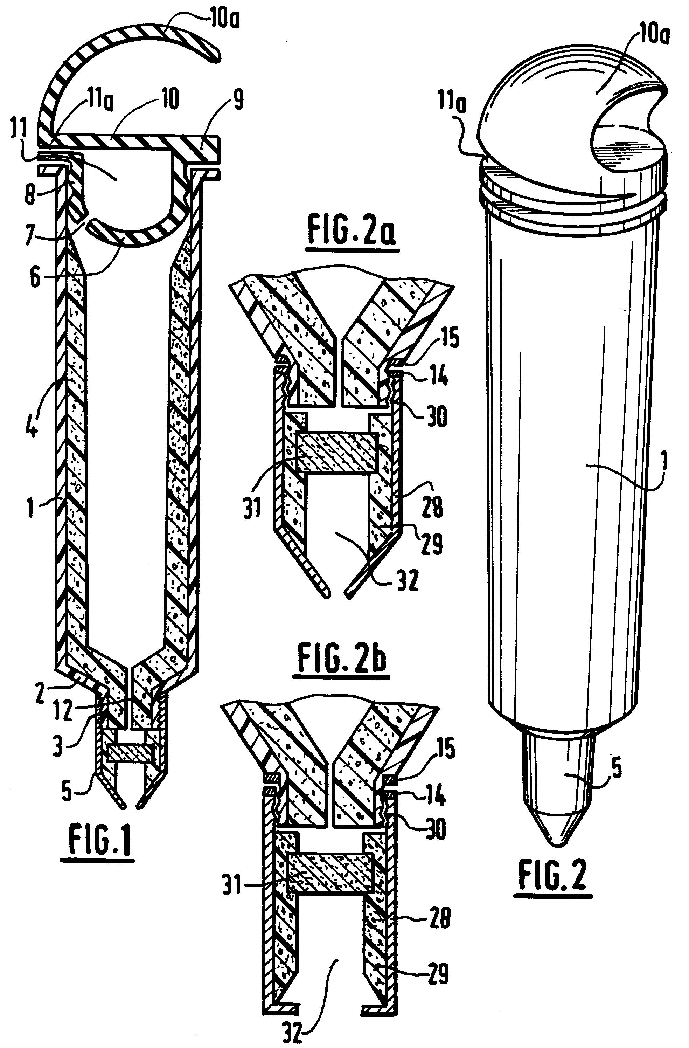

A la figure 1, il est représenté un mode de réalisation du dispositif cryogénique selon l'invention destiné à l'usage unique.

Le réservoir 1, constitué d'une matière plastique telle que le polypropylène, a une forme cylindrique.

A son extrémité inférieure, il adopte une forme conique 2 et se termine par une extrémité cylindrique 3 dont la surface externe est rainurée en pas de vis.

La hauteur de la partie cylindrique 1 est soit de 50 mm (millimètres), soit de 75 mm, soit de 100 mm, soit de 150 mm, soit de 200 mm. L'épaisseur de sa paroi est de l'ordre de 1 à 2 mm. Le diamètre du cylindre est de 30, 40 ou 50 mm.

Le réservoir est doublé à sa partie interne par un deuxième cylindre 4, qui peut être réalisé en polystyrène extrudé ou tout autre matériau présentant comme lui de bonnes caractéristiques de légèreté, de faible coût et d'isolation thermique, ici en l'occurrence aux très basses températures.

Ce cylindre isolant est perforé à son extrémité inférieure conique puis cylindrique, ce qui réalise un conduit 12 d'un calibre réduit de l'ordre du dixième de millimètre. Cependant, selon l'usage auquel sera destiné le dispositif et selon les matériaux utilisés, ce calibre pourra être plus important, de l'ordre de un millimètre par exemple.

L'épaisseur de ce cylindre isolant 4 est de l'ordre de 5 à 10 mm, sa partie supérieure pouvant être chanfreinée.

L'élément 5 est un embout dont l'extrémité supérieure comporte dans sa face interne, une rainure en pas de vis qui lui permet de se visser sur l'extrémité 3.

La figure 2a montre les détails de cet embout vissant. L'enveloppe 28 est en matière plastique, tel que le polypropylène, ou en matériau équivalent, d'épaisseur d'un millimètre. L'isolant 29 est en polystyrène extrudé ou matériau équivalent, dont l'épaisseur est de l'ordre de 2 mm. La surface interne du tiers supérieur comporte un pas de vis 30. Des épaulements 14 et 15 permettent une bonne étanchéité. Ils peuvent être réalisés dans un matériau étanche. Un filtre poreux 31 est encastré dans l'isolant 29. Une masse d'ouate de coton 32 remplit la partie distale de l'embout.

La hauteur de cet embout est, dans sa version la plus commune, de l'ordre de 20 mm.

On retrouve sur la figure 2b qui illustre un embout dans une variante à extrémité large, les mêmes éléments avec les mêmes références.

La figure 1 montre également en coupe, le bouchon 7 réalisé en matière caoutchouteuse et qui est composé de trois parties :
- une base 6, en forme de cupule à concavité supérieure, percé d'un orifice excentré 7 et dont les bords latéraux comportent deux bourrelets d'étanchéité 8. Sur un bord (celui qui est opposé à l'orifice), une zone charnière 9 relie cette base à un volet 10 de fermeture d'une chambre 11 normalement en communication avec l'atmosphère par un évent 11a, le volet étant surmonté d'un doigtier 10a destiné à recevoir un doigt, de telle sorte que lorsque la pression du doigt rabat le volet, cela crée l'occlusion de la chambre de compression 11, entraînant par le biais de l'orifice 7 une augmentation de pression de la phase gazeuse du réfrigérant qui se trouve dans le réservoir et par là même un écoulement de la phase liquide par le conduit 12, puis par l'embout applicateur.

La figure 2 représente une vue externe en perspective, du dispositif dans sa conception destinée à l'usage unique, avec son réservoir 1, son embout applicateur 5 et son bouchon régulateur de pression.

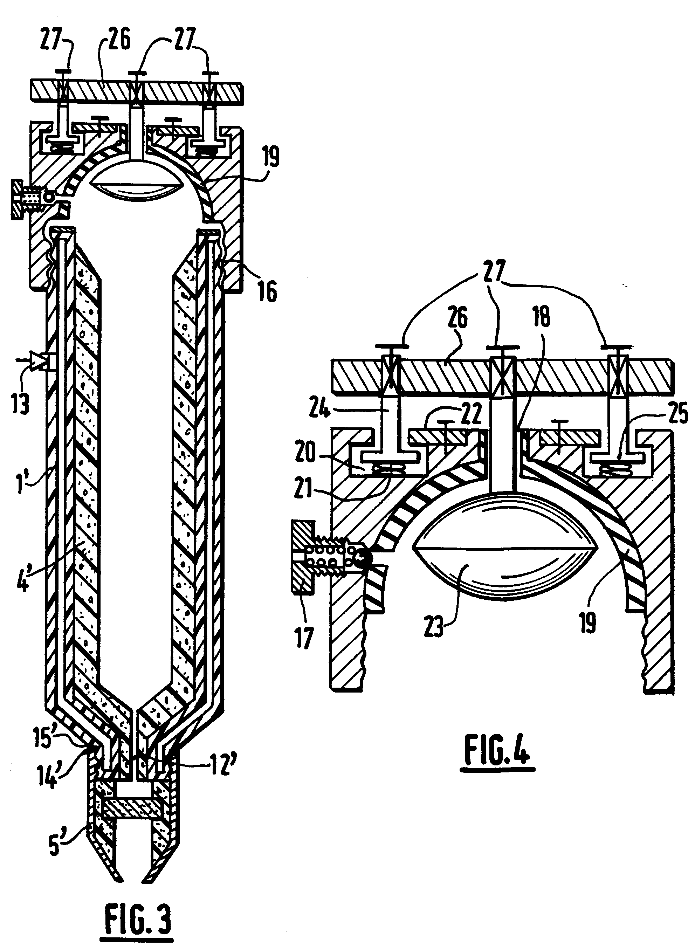

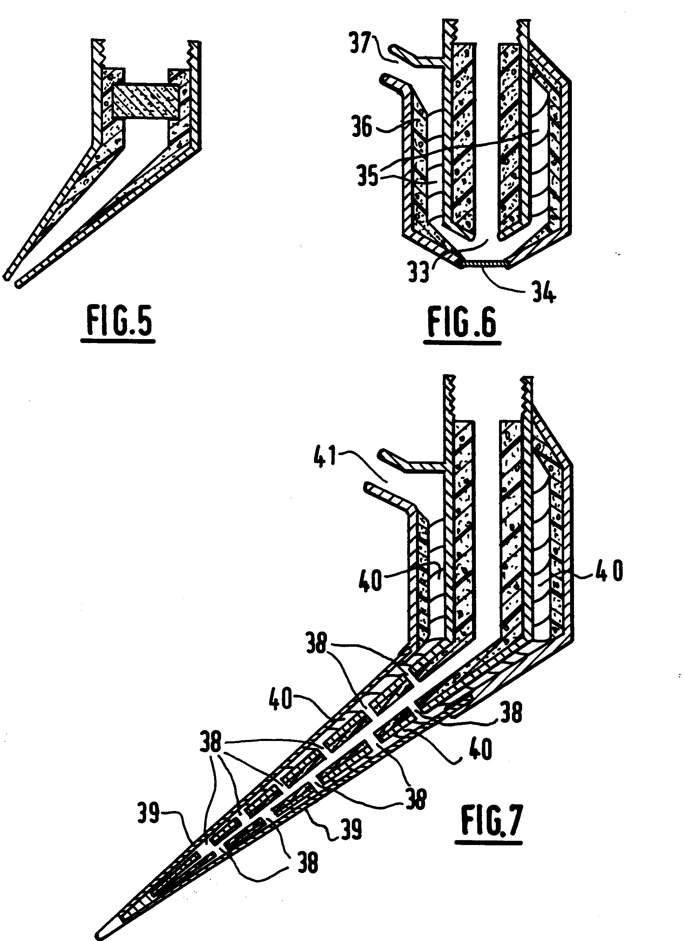

Aux figures 3 et 4, on a représenté par une coupe longitudinale un dispositif destiné à être utilisé plusieurs fois et stérilisé.

Le réservoir 1' est réalisé dans un matériau résistant et isolant thermique. A titre d'exemple, il peut être réalisé en acier inoxydable, en double paroi avec vide d'air établi au travers d'une valve 13. Une version en matière plastique stérilisable à la chaleur ou chimiquement au moyen par exemple du glutaraldéhyde,peut être prévue.

On retrouve une tunique intérieure 4' concentrique, encastrable, réalisée en matériau isolant tel que le polystyrène extrudé et pouvant être remplacée en cas d'usure ou pour fournir un calibre de l'orifice d'évacuation 12' différent.

L'embout vissant 5' s'adapte sur l'extrémité inférieure du réservoir, de manière étanche avec des joints 14' et 15' en matière supportant la stérilisation à la chaleur, telle que certains silicones.

Le bouchon vissant, non jetable et stérilisable, s'adapte sur la partie 16 supérieure et filetée du réservoir.

Il comporte deux parties :
- une partie, fixe après vissage, qui comporte :
   . une valve de sécurité 17,
   . un évent 18,
   . un joint d'étanchéité et d'isolation thermique 19 formant siège de clapet autour de l'évent 18, et
   . une cage annulaire 20 contenant quelques ressorts 21, sertis sur la base de la cage. Le toit de cette cage est constitué dans sa partie interne, d'une bague annulaire amovible et vissée 22;
- une partie mobile, composée :
   . d'une soupape d'étanchéité 23,
   . d'un cylindre 24 dont l'extrémité inférieure est une embase 25, reposant sur les ressorts 21 et retenue dans la cage par la bague 22, et
   . d'un poussoir 26, solidaire de la soupape 23 et du cylindre 24 et maintenu dans cette position par des vis 27.

Dans cette version, le bouchon vissant est maintenu en position fermée par les ressorts 21 et par la pression qu'exerce l'azote gazeux sur la soupape d'étanchéité 23.

L'azote perle alors en permanence.

Lorsque l'on désire arrêter l'écoulement de l'azote liquide, la pression sur le toit 26 entraîne la libération de la phase gazeuse et donc, par la diminution de pression, l'arrêt de l'écoulement par l'embout applicateur 5'.

La figure 5 est une coupe longitudinale d'un embout applicateur dans une variante coudée, de l'embout vu dans la figure 2a, qui permet le traitement de surfaces verticales.

La figure 6 est une coupe longitudinale d'un embout applicateur indirect. Dans cette variante, l'azote liquide s'écoule librement par l'orifice 33, il entre en contact avec une paroi métallique fine 34 (en aluminium par exemple), se vaporise à son contact puis est évacué par un conduit hélicoïdal 35, ménagé dans la paroi isolante externe 36, jusqu'à un évent 37.

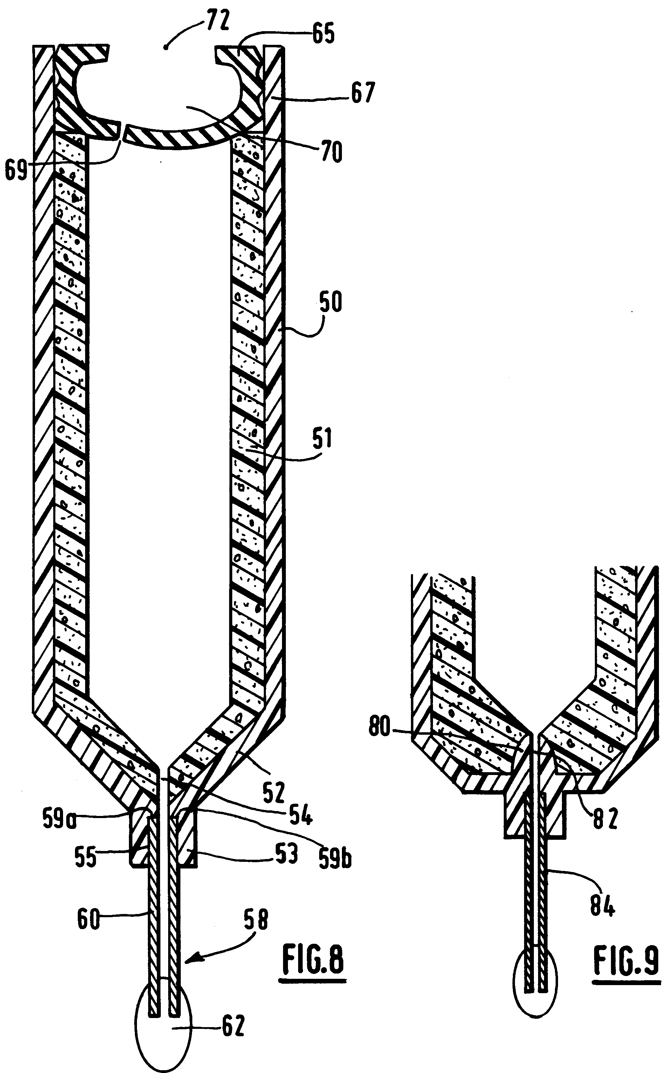

La figure 7 est une coupe longitudinale d'une variante de l'embout applicateur indirect, long et coudé, dans lequel l'azote liquide s'écoule librement par des orifices 38 de tailles croissantes de la partie proximale vers la partie distale, se vaporise au contact de la paroi métallique 39, puis est évacué par un conduit hélicoïdal 40, limité par la paroi métallique 39, jusqu'à un évent 41.

Cet embout mesure 15 mm de diamètre à son extrémité proximale et 3 mm à son extrémité distale. Cette structure permet d'envisager de longs embouts allant jusqu'à 500 mm.

La figure 8 illustre un autre mode de réalisation du dispositif selon l'invention du type comprenant deux pièces : un réservoir réutilisable et un embout à usage unique,jetable, encastré dans une extrémité du réservoir.

Le réservoir 50 est de forme cylindrique et est réalisé en matériau plastique tel que le polypropylène. Il peut être également formé d'une double enveloppe avec vide d'air à l'intérieur. Il est doublé intérieurement par un matériau isolant disposé sous la forme d'un second cylindre 51 appliqué contre la paroi interne du réservoir. Ce matériau isolant est par exemple du polystyrène extrudé.

La partie distale 52 du réservoir 50 est de forme conique puis cylindrique à son extrémité 53.

Un canal ou conduit 54 d'écoulement est prévu dans la partie distale conique du second cylindre 51 et partiellement dans le haut de l'extrémité cylindrique 53 du réservoir 50, par perforation.

Le diamètre de ce canal est de l'ordre de 1 millimètre.

Un espace de forme cylindrique 55, de diamètre plus important, est prévu en prolongement du canal 54 sur le même axe, dans la partie restante de l'extrémité cylindrique 53 du réservoir. Le diamètre de cet espace est de l'ordre de 3 à 4 millimètres.

L'espace 55 de forme cylindrique constitue un logement où va venir s'encastrer l'élément ou embout d'application 58. Des butées 59a et 59b sont prévues pour bloquer l'embout 58 introduit dans le logement 55.

Contrairement aux modes de réalisation décrits précédemment, l'embout 58 est fixé à l'intérieur du réservoir, par simple introduction de l'embout dans l'espace ou logement 55.

Cet embout 58 comprend une tige creuse 60 en matériau plastique du type polypropylène et un moyen formant filtre ou corps poreux 62. Le corps poreux 62 est enfilé sur une extrémité de la tige 60 et est alors située à l'extérieur du dispositif. Ce corps poreux est constitué notamment de fibres cellulosiques telles que par exemple de la ouate de coton.

Le diamètre externe de la tige creuse 62 est sensiblement égal à celui du logement 55 et son diamètre interne varie dans l'intervalle allant de 1 à 2 millimètres.

L'embout 58 est stérile et n'est utilisable qu'une fois. Il a une capacité de filtration et de rétention ou d'accumulation du fluide cryogénique.

Dans ce mode de réalisation, la tige creuse 60 forme le canal de sortie du liquide réfrigérant.

Le dispositif comporte également un bouchon 65 obturateur encastré dans la partie proximale 67 du réservoir 50. Ce bouchon est de préférence en matériau de type caoutchouc. 11 comporte un orifice 69 excentré. Le bouchon 65 comprend une chambre 70 ouverte sur l'extérieur par une ouverture ou évent 72 obturable par l'extrémité d'un doigt. De ce fait, la simple application d'un doigt permet d'actionner le dispositif, donc d'entraîner l'écoulement du liquide réfrigérant, et de réguler l'ouverture et la fermeture de l'évent 72, donc de moduler la quantité de liquide réfrigérant appliquée.

Ce dispositif ne nécessite donc pas de moyens supplémentaires prévus sur le bouchon, pour régler la section de l'évent. Il est de conception très simple et fonctionne parfaitement.

Il est à noter que la connexion ou encore la zone où l'embout 58 est encastré dans le logement 55, est suffisamment étanche.

Cette étanchéité peut s'expliquer par le gel de l'humidité de l'air qui se situe dans l'environnement de cette connexion, qui consolide la liaison mécanique de l'embout encastré dans l'extrémité du réservoir, et par une dilatation des matériaux formant le réservoir et l'embout dans cette zone, au contact de l'azote liquide.

La figure 9 illustre une variante de la zone où l'embout est fixé sur le réservoir du dispositif décrit ci-dessus et représenté à la figure 8.

Dans cette variante, le réservoir est moulé de telle manière à former une partie protubérante 80 orientée vers l'intérieur du réservoir et disposée sur l'axe du canal ou conduit 82. Le canal 82 est de ce fait perforé dans la partie protubérante 80. L'espace formant le logement où est encastré l'embout 84, est similaire à celui décrit pour le mode de réalisation représenté à la figure 8.

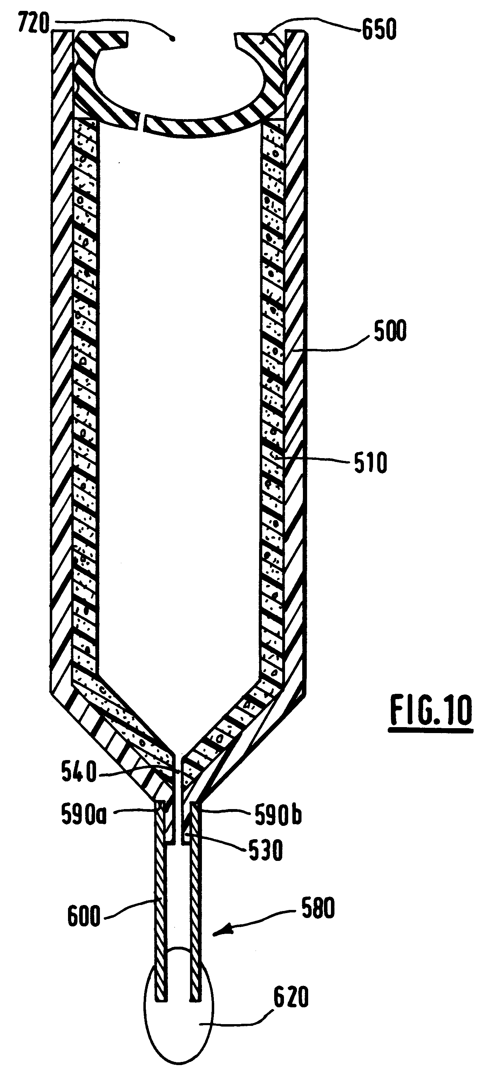

La figure 10 illustre encore un autre mode de réalisation où, à la différence du mode de réalisation représenté à la figure 8, l'extrémité 530 du réservoir 500 est encastrée dans l'extrémité proximale de l'embout d'application.

L'extrémité 530 est perforée de manière à comporter le canal 540 d'écoulement du fluide sur son axe central.

L'embout d'application 580 vient se fixer sur cette extrémité 530, en s'emboîtant autour de celle-ci et en étant bloqué par les butées 590a et 590b.

Cet embout d'application se compose également d'une tige creuse 600 et d'un corps poreux ou moyen formant filtre 620.

Le dispositif selon l'invention peut être présenté sous la forme d'un kit ou coffret comprenant l'ensemble des éléments nécessaires pour construire le dispositif selon l'invention, à savoir : un élément formant réservoir et un bouchon, réutilisables, et une série d'embouts stériles, à usage unique et jetable. Le praticien monte alors le dispositif et l'utilise avec un embout donné pour chaque geste. Plusieurs formes d'embout peuvent être proposées pour les différents traitements et zones de traitement. Le praticien n'a alors qu'à retirer l'embout utilisé et le remplacer par un autre embout.

Le kit ou coffret peut également comprendre accessoirement un moyen pour permettre le remplissage du réservoir du dispositif, par exemple un godet muni d'un bec verseur ou encore un entonnoir.

L'invention comprend tous les moyens équivalents ou secondaires qui n'ont pas été décrits dans les modes de réalisation illustrant l'invention et qui sont à la portée de l'homme du métier.

## Revendications

**1)** Dispositif à main pour le traitement cryogénique d'un corps, comportant un conteneur pour un fluide cryogénique et une extrémité d'application, caractérisé en ce que le conteneur est en forme de réservoir isotherme (1,1',50,500) comportant à l'une de ses extrémités une large ouverture de remplissage obturable par un bouchon (6 à 10,65,650) tandis que son autre extrémité est traversée par un canal ou conduit (12,12',54,540) d'écoulement du liquide. cette autre extrémité étant équipée d'un embout d'application (5,5',58,84,580) adapté, le bouchon d'obturation du réservoir étant pourvu d'un évent (11a,72,720) d'échappement de la fraction vaporisée du liquide réfrigérant et comportant le cas échéant, des moyens (10, 10a) pour régler la section de cet évent.

**2)** Dispositif selon la revendication 1, caractérisé en ce que l'extrémité du réservoir (1,1',50,500) comportant le canal ou conduit (12,54) est équipée de moyens pour recevoir un embout (5,5',58,84,580) d'application choisi parmi une pluralité d'embouts de formes et de tailles différentes.

**3)** Dispositif selon la revendication 1 ou la revendication 2, caractérisé en ce que chaque embout (5,5',58,84,580) est traversé par un canal de sortie du liquide réfrigérant au travers d'un corps poreux et filtrant (31,62,620) de rétention.

**4)** Dispositif selon la revendication 3, caractérisé en ce que l'embout (5) comporte une paroi externe (34, 39) définissant au moins une zone d'application dont la face interne limite un canal de circulation (35, 40) du liquide réfrigérant provenant d'un conduit central dans l'embout et s'échappant sous forme gazeuse par un évent (37, 41) de l'embout ménagé dans la paroi externe de ce dernier en dehors de la zone d'application (34, 39).

**5)** Dispositif selon l'une quelconque des revendications précédentes, caractérisé en ce que le réservoir comporte une paroi externe (1,1',50,500) mince et une paroi interne (4,4',51,510) extractible épaisse dans laquelle est ménagé le canal ou conduit (12,12',54,540) de sortie du liquide réfrigérant.

**6)** Dispositif selon la revendication 5, caractérisé en ce que la paroi externe (1'), est à double enveloppe avec vide d'air à l'intérieur.

**7)** Dispositif selon l'une quelconque des revendications précédentes. caractérisé en ce que les moyens de réglage de la section de l'évent (18) prévu dans le bouchon sont normalement fermés et possèdent des organes (26) pour leur actionnement manuel à l'ouverture.

**8)** Dispositif selon la revendication 7. caractérisé en ce que ces moyens sont constitués par un clapet (23) coopérant avec un siège (19) ménagé dans le bouchon et dont l'organe de manoeuvre est formé par un poussoir (26) situé au-delà du bouchon et solidaire du clapet par une tige de guidage.

**9)** Dispositif selon l'une quelconque des revendications 1 à 5, caractérisé en ce que les moyens de réglage de la section de l'évent (11a) prévu dans le bouchon sont normalement ouverts et possèdent des organes (10, 10a) de leur actionnement à la fermeture.

**10)** Dispositif selon la revendication 9, caractérisé en ce que le bouchon est en matière souple et élastique et comporte une paroi supérieure (10) formant volet d'obturation de l'évent (11a) ménagé entre elle et une paroi périphérique (8) du bouchon, cette paroi supérieure étant surmontée d'un doigtier (10a) pour le logement de l'extrémité de l'index de l'opérateur.

**11)** Dispositif selon l'une quelconque des revendications précédentes. caractérisé en ce que l'embout est coudé, la partie distale étant effilée en forme de pointe.

**12)** Dispositif selon l'une des revendications 1 à 3. 5 et 6, caractérisé en ce que l'embout (58,84,580) comprend une tige creuse (60,600) et un corps poreux de rétention (62,620), et est fixé sur l'extrémité (53,530) du réservoir (50,500), par encastrement.

**13)** Dispositif selon l'une des revendications 1 à 6, 11 et 12, caractérisé en ce que le bouchon (65,650) d'obturation du réservoir ne comporte pas de moyens pour régler la section de cet évent, cette fonction pouvant être remplie par un doigt de l'utilisateur.

**14)** Kit pour construire le dispositif selon l'une des revendications précédentes. caractérisé en ce qu'il comprend un réservoir isotherme, un bouchon muni d'un évent et destiné à venir se fixer par vissage ou encastrement sur une extrémité du réservoir, et un ou plusieurs embouts d'application de préférence à usage unique, se fixant sur l'autre extrémité du réservoir, par vissage ou encastrement ou tout autre moyen.
